# EUROPEAN PATENT APPLICATION

(11) **EP 1 797 773 A1**
(43) Date of publication of application: **20.06.2007**
(21) Application number: 05112316.4
(22) Date of filing: 16.12.2005
(51) Int. Cl.: A23L 1/015, A23L 1/314, A23L 1/318, A23B 4/24

(54) **Aqueous potassium lactate solution**

(71) Applicant: PURAC Biochem BV, 4206 AC Gorinchem (NL)
(72) Inventor: Van Dijk, Lonneke, 3061 VE, Rotterdam (NL); Rodriquez Longarela, Gema, 4207 VC, Gorinchem (NL); Sinnema, Mirjam, 3011 DC, ROTTERDAM (NL); Fahlin, Kathleen, BLAIR, NE 68008, NE Nebraska (US)
(74) Representative: Beetz, Tom

(57) **Abstract**

The invention relates to a method for making a potassium lactate solution, with improved flavor and/or odor properties wherein a first potassium lactate solution having a concentration less than 65% is concentrated by evaporation to a solution having a potassium lactate concentration from 65 to 85%. The method may be preceded or followed by treatment of the solution with active carbon.

## Description

The invention relates to an aqueous potassium lactate solution, to a method for making the same, and to a method for removing bad flavor and/or bad odor from aqueous potassium lactate solutions.

Aqueous potassium lactate solutions are known in the art and are commercially available. Potassium lactate is a product that is mainly used in the meat industry. It is used for the same application as sodium lactate, namely for shelf life extension and safety enhancement. The use of potassium lactate is more and more preferred since it is believed that sodium lactate can cause hypertension. A disadvantage of such aqueous potassium lactate solutions is their undesirable flavors and odors, particularly causing a bitter aftertaste.

In EP 1385815 a process for the removal of undesirable flavors and odors from potassium lactate was described by treating the potassium lactate solution with active carbon. The resulting potassium lactate is less bitter and has a more favorable flavor and odor profile and is thus accepted by a large part of the population. The potassium lactate is treated in the form of an aqueous solution. The concentration of potassium lactate in the solution to be treated is 40 to 80% by weight based on the combined weight of water and potassium lactate and the process can be carried out continuously or batch wise.

During the production of lactic acid by fermentation, normally neutralizing agents are used to neutralize the fermentation broth. In, for instance, GB 907,321 it has been described that calcium sulfate is used as neutralizing agent so that calcium lactate is formed. Afterwards, the calcium ions are via ion exchange replaced by hydrogen but can also be replaced by sodium or potassium ions. Solutions thus obtained are not suitable for food applications since they contain substantial amounts of impurities such as ethanol, acetaldehyde, polysaccharides, diacetyl, 2-butanone, 2,3-pentanedione, and 2-butenal which cause bad odor and bad flavor. The free lactic acid, not its potassium salt, may be subjected to further purification and concentration.

A potassium lactate product that is purified according to prior art methods is, for instance, PURASAL HiPure® P, which is commercially obtainable as a 60% solution by Purac Biochem BV, the Netherlands. When converting lactic acid to potassium lactate theoretically, higher concentrations than 60% can be obtained in this manner, but practically not. The reaction is highly exothermic and any attempt to make higher concentrations would inevitably lead to unacceptable unsafe reaction conditions, and due to the excessive high temperatures to formation of side products.

The active carbon method according to the prior art has several drawbacks. Firstly, the active carbon treatment is expensive due to its elaborated and time-consuming regeneration, which adds considerable to the costs of the product that needs to be cheap for its intended use. Secondly, as the quality of the regenerated carbon of this process fluctuates, the quality of the potassium lactate also fluctuates. Thirdly, the flavor and odor properties of this product are still susceptible to improvement.

It is therefore an objective of the present invention to provide a simpler method that provides aqueous potassium lactate solutions with improved organoleptic properties, and wherein carbon, if used, can more easily be regenerated.

To this end the invention pertains to a method for making a potassium lactate solution, with improved flavor and/or odor properties wherein a first potassium lactate solution having a concentration less than 65% is concentrated by evaporation to a solution having a potassium lactate concentration from 65 to 85%.

The product quality of the product obtained by the present method was improved, as key aroma compounds appear to be removed from the product. In order to obtain higher quality products still having 60% potassium lactate, the first potassium lactate solution can be concentrated to a certain concentration between 65 and 85% and then diluted again to 60%.

Since commercially available potassium lactate solutions commonly have a potassium lactate concentration of about 60% it is preferred to use such solutions as the first (starting) potassium lactate solution.

Evaporation can be performed using any method for evaporation, such as distillation, flash distillation, wiped film distillation, short path distillation, vacuum distillation, rotary evaporation, spinning cone column evaporation, and the like. Sometimes it is preferred to perform the distillation in multiple, for instance two, separate distillation steps. In a first distillation step the first potassium lactate solution can be concentrated, for example, from 60% to 67%, and in a second distillation step this partially concentrated potassium lactate solution can be further concentrated from said 67% to, for example, 78%. If two distillation steps are used, it is further preferred to perform these steps in two different distillation units, but the evaporation can also take place in one apparatus because most evaporation apparatuses can be used as multi-step evaporator. As a suitable example the use of a plate evaporator is mentioned, wherein the liquid is pumped between thin plates with the heating medium on the mating surfaces, after which the product is evaporated with the vapor generated forming a high velocity core.

As explained, if possible, it is of advantage replacing the active carbon step by an evaporation step. However, due to coloring of the end product such step cannot be deleted always. Thus if the product should be decolorized, an active carbon step may still be necessary. Such step can be performed prior to the evaporation step, for instance by using PURASAL HiPure P as the starting material, or alternatively the active carbon treatment can be performed after the evaporation step. It was found that such combined active carbon/evaporation method provided the highest quality end product. When performed prior to evaporation, it was found that a substantial improvement regarding taste and odor was achieved. When performed after evaporation, it was found that the regenerated carbon, and thus the resulting potassium lactate, showed less quality fluctuations.

It is a further objective of the invention to provide a method for removing bad flavor and/or bad odor from a first potassium lactate solution having a potassium lactate concentration less than 65% by evaporating said solution to a potassium lactate solution having a concentration from 65 to 85%, and optionally diluting said solution again to below 65%, for instance to about 60%.

It is also an objective of the invention to provide an aqueous potassium lactate solution having a potassium lactate concentration from 65 to 85%, and which is free from or at least poor in bad flavor and/or odor. Such product is obtainable by the above evaporation method with or without the additional active carbon purification step. It was further found that solutions thus obtained contained less than 5 ppm of residual ethanol.

Preferred potassium lactate solutions have a potassium lactate concentration of 72 to 82%, more preferably 76 to 82%. A particularly useful concentration is more than 80% to less than 82%. Concentrations higher than 85% lead to viscous solutions that are less suitable and more difficult to handle in meat production where low temperatures such as 4° C are used, but are still suitable for transport. The concentrated solutions as obtained according to the invention may be diluted to any diluted solution, for instance to a solution containing 60% potassium lactate. The higher concentration solutions, however, have the additional advantage that bulk amounts can more easily be transported because these need less container space for the same amount of potassium lactate.

The invention is further illustrated by the following non-limitative examples.

### Experimental

Five samples containing 60% of potassium lactate were evaporated by SPD (short path distillation) to obtain a concentrated solution.

The temperature of the heating oil was varied in order to obtain different concentrations. Table 1 gives the final concentration of the samples.

**Table 1. Concentration of the samples**

| Sample | Tₒᵢₗ [°C] | Concentration [wt.%] |
|---|---|---|
| 1 | 100 | 67.1 |
| 2 | 115 | 70.6 |
| 3 | 145 | 78.1 |
| 4 | 160 | 82.1 |
| 5 | Reference* | 60.0 |

| | | |
|---|---|---|
| *PURASAL HiPure® P; ex Purac America Inc., USA | | |

### GC-MS results

The relative areas of key aroma compounds found by GC-MS are given in Table 2. The concentrated potassium lactate solutions were diluted to 60%, prior to analysis.

**Table 2. Relative areas of key aroma compounds**

| Concentration (%) | 60* | 67.1 | 70.6 | 78.1 | 82.1 |
|---|---|---|---|---|---|
| | | | | | |
| Acetaldehyde | 43.38 | 2.20 | 1.54 | 1.00 | 1.45 |
| Ethanol | 53.64 | 5.74 | 1.92 | 1.00 | 6.93 |
| Diacetyl | 3.46 | 1.00 | 1.03 | 1.34 | 1.92 |
| 2-Butanone | 37.21 | 1.00 | 1.18 | 1.06 | 1.14 |
| 2,3-Pentanedione | 7.79 | 1.22 | 1.09 | 1.00 | 1.51 |
| 2(E)-Butenal | 8.12 | 1.63 | 1.41 | 1.00 | 1.44 |
| Total | 153.60 | 12.78 | 8.17 | 6.40 | 14.39 |

| | | | | | |
|---|---|---|---|---|---|
| * reference = PURASAL HiPure P | | | | | |

### Taste and odor

Odor and taste were tested by a sensory panel. Four samples were selected based on the GC-MS results. These samples were compared with a sample P-Lac®, 60% potassium lactate (ex Wilke Resources Inc., USA).

The samples for the odor test were diluted to 60% dry solids. The samples for taste were tested by diluting the samples to 1.12% dry solids. 6 Panel members judged the samples on taste and 4 panel members judged the samples on odor. The samples were ranked from 1 (low) to 5 (high).

Tables 3 and 4 show the results of the odor and taste tests, respectively.

**Table 3. Results of odor**

| Sample | Concentration (wt.%) | Average ranking* |
|---|---|---|
| 1 | 67.1 | 2.0 |
| 3 | 78.1 | 2.5 |
| 5# | 60.0 | 3.0 |
| P-Lac® | 60.0 | 4.0 |

| | | |
|---|---|---|
| * 1:low intensity, 5:high intensity # PURASAL HiPure P (reference) | | |

**Table 4. Results of taste: intensity and bitterness**

| Sample | Concentration (wt%) | Average ranking Intensity* | Average ranking Bitterness* |
|---|---|---|---|
| 1 | 67.1 | 1.8 | 2.0 |
| 3 | 78.1 | 2.0 | 2.2 |
| 5# | 60.0 | 4.5 | 4.2 |
| P-Lac® | 60.0 | 3.2 | 3.0 |

| | | | |
|---|---|---|---|
| * 1:low intensity; 5:high intensity 5 panel members ranked the samples on bitterness # PURASAL HiPure® P (reference) | | | |

## Claims

1. A method for making a potassium lactate solution, with improved flavor and/or odor properties wherein a first potassium lactate solution having a concentration less than 65% is concentrated by evaporation to a solution having a potassium lactate concentration from 65 to 85%.

2. The method according to claim 1 wherein the first potassium lactate solution has a potassium lactate concentration of about 60%.

3. The method according to claim 1 or 2 wherein the evaporation is performed in two steps, optionally in two distillation apparatuses.

4. The method according to any one of claims 1 to 3 wherein the evaporation is performed by a plate evaporation process.

5. The method according to any one of claim 1 to 4 wherein the evaporation is preceded or followed by treatment of the solution with active carbon.

6. A method for removing bad flavor and/or odor from a first potassium lactate solution having a potassium lactate concentration less than 65% by evaporating said solution to a potassium lactate solution having a concentration from 65 to 85%.

7. The method according to claim 6 wherein the evaporation is preceded or followed by treatment of the solution with active carbon.

8. A potassium lactate solution with improved flavor and/or odor properties having a potassium lactate concentration from 65 to 85%, obtainable by the method of any one of claims 1-6.

9. A potassium lactate solution with improved flavor and/or odor properties having a potassium lactate concentration from 65 to 85% and an ethanol content less than 5 ppm.

10. The potassium lactate solution of claim 8 or 9, wherein the potassium lactate concentration is 72 to 82%, preferably 76 to 82%, more preferably more than 80 to less than 82%.
